# EUROPEAN PATENT APPLICATION

(11) **EP 3 498 841 A1**
(43) Date of publication of application: **19.06.2019**
(21) Application number: 17860359.3
(22) Date of filing: 05.09.2017
(51) Int. Cl.: C12N 15/09, C12N 1/21

(54) **OBLIGATELY ANAEROBIC ACETIC ACID-PRODUCING MICROORGANISM, AND RECOMBINANT MICROORGANISM**

(30) Priority: 13.10.2016 JP 2016201947
(71) Applicant: Sekisui Chemical Co., Ltd., Osaka-shi, Osaka 530-8565 (JP)
(72) Inventor: FUKUNISHI Kana, Tsukuba-shi Ibaraki 300-4292 (JP); FURUTANI Masahiro, Tsukuba-shi Ibaraki 300-4292 (JP); KAWABATA Kazufumi, Tsukuba-shi Ibaraki 300-4292 (JP)
(74) Representative: Ter Meer Steinmeister & Partner
(86) International application number: PCT/JP2017/031922
(87) International publication number: WO 2018/070141

(57) **Abstract**

Provided is a modified obligately anaerobic acetogen in which activity of at least one enzyme selected from the group consisting of a restriction enzyme, a modification enzyme, and a recognition enzyme constituting a type I restriction modification system enzyme is deleted or suppressed. Preferably, the activity of at least the restriction enzyme is deleted or suppressed. The obligately anaerobic acetogen is preferably a *Clostridium* bacterium or a *Moorella* bacterium, and particularly preferably *Clostridium ljungdahlii*.

<110>  Sekisui Chemical Co., Ltd.

<120>  偏性嫌気性酢酸生成微生物、並びに、組換え微生物

<130>  17P00956

<150>  JP 2016-201947
<151>  2016-10-13

<160>  5

<170>  PatentIn version 3.1

<210>  1
<211>  1318
<212>  PRT
<213>  Clostridium ljungdahlii

<400>  1

Met Tyr Phe Asp Lys Glu Thr Asp Phe Glu Glu Ala Val Ile Lys Ala
1               5                   10                  15

Leu Ile Glu Cys Gly Trp Glu Ser Glu Val Ile Arg His Pro Ser Glu
            20                  25                  30

Lys Asp Leu Ile Lys Asn Trp Ala Asn Ile Leu Phe Asn Asn Asn Arg
        35                  40                  45

Gln Arg Asp Arg Leu Asn Glu Cys Pro Leu Thr Asp Gly Glu Met Gln
    50                  55                  60

Gln Ile Met Glu Gln Ile Lys Leu Leu Gln Ser Pro Ile Lys Leu Asn
65                  70                  75                  80

Gly Phe Ile Asn Gly Lys Ser Val Ser Ile Lys Arg Asp Asn Pro Asp
                85                  90                  95

Asp Lys Glu His Phe Gly Lys Glu Val Ser Leu Lys Ile Tyr Asp Arg
            100                 105                 110

Gln Glu Ile Ala Tyr Gly Glu Ser Arg Tyr Gln Ile Val Gln Gln Pro
        115                 120                 125

His Phe Pro Thr Lys Ser Pro Ile Leu Asn Asn Arg Arg Gly Asp Leu
    130                 135                 140

Met Leu Leu Ile Asn Gly Met Pro Val Ile His Ile Glu Leu Lys Lys
145                 150                 155                 160

Ser Gly Ile Pro Val Ser Glu Ala Tyr Asn Gln Ile Glu Lys Tyr Ser
                165                 170                 175

His Glu Gly Val Phe Arg Gly Leu Phe Ser Leu Ile Gln Val Phe Val
            180                 185                 190

Ala Met Gln Pro Glu Glu Thr Val Tyr Phe Ala Asn Pro Gly Glu Gly
        195                 200                 205

Val Lys Phe Asn Lys Ala Phe Tyr Phe His Trp Ala Asn Phe Asn Asn
    210                 215                 220

Gln Pro Ile Asn Lys Trp Asp Glu Val Ile Ser Lys Leu Leu Asn Ile
225                 230                 235                 240

Pro Met Ala His Met Ile Ile Gly Phe Tyr Thr Ile Ala Asp Thr Asp
                245                 250                 255

Glu Gly Val Leu Lys Val Met Arg Ser Tyr Gln Tyr Tyr Ala Ala Ile
            260                 265                 270

Gly Ile Ala Asp Lys Val Ala Lys Lys Asn Trp Lys Asp Asn Asn Gln
        275                 280                 285

Leu Gly Gly His Val Trp His Thr Thr Gly Ser Gly Lys Thr Met Thr
    290                 295                 300

Ser Phe Lys Ser Ala Gln Leu Ile Ala Ser Ser His Asp Ala Asp Lys
305                 310                 315                 320

Val Ile Phe Leu Val Asp Arg Lys Glu Leu Gly Ile Gln Ser Leu Lys
                325                 330                 335

Glu Tyr Arg Ser Phe Ala Asn Glu Ser Glu Ser Val Gln Ala Thr Glu
            340                 345                 350

Asn Thr Asn Ile Leu Ile Ser Lys Leu Lys Ser Asp Asp Leu Asp Asn
        355                 360                 365

Thr Leu Ile Val Thr Ser Ile Gln Lys Leu Ser Asn Ile Ser Glu Glu
    370                 375                 380

Ala Gln Glu Leu Asn Glu Ala Asp Leu Lys Ile Ile Gln Ser Lys Arg
385                 390                 395                 400

Ile Ala Ile Ile Ile Asp Glu Cys His Arg Ser Thr Phe Gly Glu Met
                405                 410                 415

Leu Thr Asn Ile Lys Asn Thr Phe Lys His Ser Ile Ile Phe Gly Phe
            420                 425                 430

Thr Gly Thr Pro Ile Gln Asp Val Asn Ile Lys Lys Asp Ser Thr Thr
        435                 440                 445

Pro Thr Ile Phe Gly Asp Glu Ile His Arg Tyr Thr Leu Ala Asp Gly
    450                 455                 460

Ile Arg Asp Lys Asn Val Leu Gly Phe Asp Pro Tyr Met Val Lys Ile
465                 470                 475                 480

Tyr Asp Asp Ser Asp Leu Arg Gln Ala Val Ala Leu His Lys Ala Lys
                485                 490                 495

Ala Ala Thr Val Glu Glu Val Met Gly Asp Pro Lys Lys Glu Lys Met
            500                 505                 510

Phe Tyr Lys Tyr Met Asp Ser Ser Gln Val Lys Met Tyr Gly Glu Gln
        515                 520                 525

Val Asp Gly Lys Trp Val Ala Gly Ile Glu Asp Phe Ile Pro Asn Glu
    530                 535                 540

Gln Tyr Gln Thr Ala Glu Tyr Arg Asn Gly Val Ile Ala Asp Ile Lys
545                 550                 555                 560

Lys Lys Trp Thr Ile Tyr Ser Arg Gly Arg Lys Phe His Ala Ile Phe
                565                 570                 575

Ala Thr Ser Asn Ile Pro Glu Ala Val Val Tyr Tyr Arg Leu Met Lys
            580                 585                 590

Ala Glu Met Pro Asn Leu Ser Ile Thr Ala Met Phe Asp Pro Ser Ile
        595                 600                 605

Asp Asn Glu Gly Gly Gly Ser Leu Glu Lys Glu Asp Gly Ile Val Glu
    610                 615                 620

Ile Leu Glu Asp Tyr Lys Ala Lys Phe Ser Gln Ser Phe Thr Ile Lys
625                 630                 635                 640

Ser Tyr Asp Lys Phe Arg Lys Asp Val Ser Leu Arg Leu Ala His Lys

645                 650                 655

Lys Pro Tyr Glu Arg Ile Thr Pro Asp Glu Gln Ile Asp Ile Leu Ile
            660                 665                 670

Val Val Asn Gln Met Leu Thr Gly Phe Asp Ser Lys Trp Val Asn Thr
        675                 680                 685

Leu Tyr Leu Asp Lys Val Met Glu Tyr Glu Asn Leu Ile Gln Ala Phe
    690                 695                 700

Ser Arg Thr Asn Arg Leu Phe Asn Val Ala Asp Lys Pro Phe Gly Ile
705                 710                 715                 720

Ile Lys Tyr Tyr Arg His Pro Asn Thr Met Glu Lys Asn Ile Glu Ala
                725                 730                 735

Ala Val Lys Ala Tyr Ser Gly Asp Ile Pro Thr Gly Leu Phe Val Asp
            740                 745                 750

Lys Leu Pro Asn Asn Leu Arg Gln Met Asn Ser Leu Phe Asp Glu Ile
        755                 760                 765

Gln Ile Leu Phe Lys Asn Ala Ser Ile Ser Asn Phe Glu Arg Leu Pro
    770                 775                 780

Asp Glu Ser Ser Val Lys Ala Lys Phe Ala Lys Gln Phe Lys Gln Phe
785                 790                 795                 800

Ser Thr His Leu Glu Ala Ala Thr Ile Gln Gly Phe Ile Trp Ser Gln
                805                 810                 815

Lys Thr Tyr Ser Asp Glu Asn Gly Thr Ser Ile Val Met His Leu Asp
            820                 825                 830

Glu Met Thr Tyr Leu Ile Leu Leu Ala Arg Tyr Met Glu Leu Ala Lys
        835                 840                 845

Gly Gly Gly Gly Gly Arg Ser Gly Asp Val Pro Tyr Glu Ile Asp Thr
    850                 855                 860

His Ile Thr Glu Tyr Asp Thr Glu Lys Ile Asp Ala Asp Tyr Met Asn
865                 870                 875                 880

Ser Arg Phe Glu Lys Phe Leu Lys Leu Ile Glu Ser Glu Tyr Asp Ala
                885                 890                 895

Glu Ser Leu Asp Lys Thr Leu Lys Glu Leu His Lys Ser Phe Ser Met
            900                 905                 910

Leu Ser Gln Glu Glu Gln Lys Tyr Ala Ile Ile Phe Ile Arg Asp Ile
        915                 920                 925

Gln Ala Gly Asn Ile His Val Ile Pro Gly Lys Ser Phe Arg Asn Tyr
    930                 935                 940

Ile Ser Gln Met Met Lys Lys Ala Glu Asn Asp Arg Ile Lys Arg Val
945                 950                 955                 960

Val Asn Arg Leu Gly Cys Tyr Glu Lys Leu Leu Arg Glu Met Leu Gln
                965                 970                 975

Arg Lys Val Thr Lys Glu Thr Ile Glu Ala His Gly Lys Phe Asp Glu
            980                 985                 990

Leu Lys Ala Thr Val Asp Asn Gln Lys Ala Arg Thr Phe Phe Ile Leu
        995                 1000                1005

Val Glu Gln Asp Asn Tyr Gln Glu Ser Arg Leu Ala Met Tyr Ile
    1010                1015                1020

Glu Gln Tyr Leu Arg Asn Phe Leu Leu Ser Gly Gly Glu Asp Pro
    1025                1030                1035

Tyr Ser Asn Val Glu Tyr Ser Glu Val Ile Arg Glu Lys Asn Glu
    1040                1045                1050

Ser Glu Tyr Asn Ala Ser Val Gly Val Val Leu Thr Glu Glu Lys
    1055                1060                1065

Met Lys Gly Lys Ser Ile Val Ser Ser Val Lys Ser Ala Thr Leu
    1070                1075                1080

Asn Asn Trp Tyr Ser Glu Ser Lys Ala Leu Ala Cys Met Leu Glu
    1085                1090                1095

Thr Asp Cys Phe Ala Tyr Val Glu Asn Lys Leu Cys Ile Tyr Asp
    1100                1105                1110

Asn Lys Tyr Ile Gln Arg Asn Gln Asn Gly Arg Met Phe Leu Thr
    1115                1120                1125

Asn Tyr Ala Lys Glu His Glu Glu Glu Cys Phe Leu Gln Phe Ile
    1130                1135                1140

Ile Asp Asn Glu Thr Gly Lys Leu His Tyr Ile Thr Leu Pro Ala
    1145                1150                1155

Ala Met Ala Ser Lys Ser Phe Asn Tyr Tyr Asp Glu Ile Asn Glu
    1160                1165                1170

Asp Leu Leu Thr Gln Tyr Gly Leu Val Asn Glu Ile Ser His Glu
    1175                1180                1185

Met Leu Val Ala Ile Asn Gly Leu Asp Phe Gly Asp Ala Leu Thr
    1190                1195                1200

Lys Leu Met Ser Lys Asn Ile Cys Asn Tyr Ser Val Arg Leu Leu
    1205                1210                1215

Arg Asp Thr Thr Gly Leu Asp Asn Arg Thr Ile Ser Asn Met Glu
    1220                1225                1230

Lys Gly Asn Asn Leu Thr Lys Ile Asn Val Ile Ser Ala Cys Leu
    1235                1240                1245

Gly Ile Gln Ile Pro Phe Arg Val Ser Asn Lys Met Leu Gln Leu
    1250                1255                1260

Ala Glu Leu Ser Leu Asn Phe Asp Leu Pro Gly Lys Lys Gly Glu
    1265                1270                1275

Glu Asn Gly Ile Tyr Asp Ser Val Leu His Leu Lys Trp Ala Thr
    1280                1285                1290

Asp Tyr Glu Asp Val Tyr Glu Glu Leu Lys Glu Gln Asn Tyr Glu
    1295                1300                1305

Tyr Leu Leu His Arg Pro Lys Lys Ile Lys
    1310                1315

<210> 2
<211> 6701
<212> DNA
<213> Artificial

<220>
<223> Artificial gene

<400>  2
gcggccgctc agtcacctcc tagctgactc aaatcaatgc gtgtttcata aagaccagtg      60

atggattgat ggataagagt ggcatctaaa acttcttttg tagacgtata tcgtttacga     120

tcaattgttg tatcaaaata tttaaaagca gcgggagctc caagattcgt caacgtaaat     180

aaatgaataa tattttctgc ttgttcacgt attggtttgt ctctatgttt gttatatgca     240

ctaagaactt tatctaaatt ggcatctgct aaaataacac gcttagaaaa ttcactgatt     300

tgctcaataa tctcatctaa ataatgctta tgctgctcca caaacaattg tttttgttcg     360

ttatcttctg gactaccctt caacttttca taatgactag ctaaatataa aaaattcaca     420

tatttgcttg gcagagccag ctcatttcct ttttgtaatt ctccggcact agccagcatc     480

cgtttacgac cgttttctaa ctcaaaaaga ctatatttag gtagtttaat gattaagtct     540

tttttaactt ccttatatcc tttagcttct aaaaagtcaa tcggattttt ttcaaaggaa     600

cttctttcca taattgtgat ccctagtaac tctttaacgg attttaactt cttcgatttc     660

cctttttcca ccttagcaac cactaggact gaataagcta ccgttggact atcaaaacca     720

ccatattttt ttggatccca gtctttttta cgagcaataa gcttgtccga atttcttttt     780

ggtaaaattg actccttgga gaatccgcct gtctgtactt ctgttttctt gacaatattg     840

acttggggca tggacaatac tttgcgcact gtggcaaaat ctcgcccttt atcccagaca     900

atttctccag tttccccatt agtttcgatt agagggcgtt tgcgaatctc tccatttgca     960

agtgtaattt ctgttttgaa gaagttcatg atattagagt aaaagaaata ttttgcggtt    1020

gctttgccta tttcttgctc agacttagca atcattttac gaacatcata aactttataa    1080

tcaccataga caaactccga ttcaagtttt ggatatttct taatcaaagc agttccaacg    1140

acggcattta gatacgcatc atgggcatga tggtaattgt taatctcacg tactttatag    1200

aattggaaat cttttcggaa gtcagaaact aatttagatt ttaaggtaat cactttaacc    1260

tctcgaataa gtttatcatt ttcatcgtat ttagtattca tgcgactatc caaaatttgt    1320

gccacatgct tagtgatttg gcgagtttca accaattggc gtttgataaa accagcttta    1380

tcaagttcac tcaaacctcc acgttcagct ttcgttaaat tatcaaactt acgttgagtg    1440

attaacttgg cgtttagaag ttgtctccaa tagtttttca tctttttgac tacttcttca    1500

cttggaacgt tatccgattt accacgattt ttatcagaac gcgttaagac cttattgtct    1560

attgaatcgt ctttaaggaa actttgtgga acaatgtgat cgacatcata atcacttaaa    1620

cgattaatat ctaattcttg gtccacatac atgtctcttc cattttggag ataatagaga    1680

tagagctttt cattttgcaa ttgagtattt tcaacaggat gctctttaag aatctgactt    1740

cctaattctt tgataccttc ttcgattcgt ttcatacgct ctcgcgaatt tttctggccc    1800

ttttgagttg tctgattttc acgtgccatt tcaataacga tattttctgg cttatgccgc 1860

cccattactt tgaccaattc atcaacaact tttacagtct gtaaaatacc ttttttaata 1920

gcagggctac cagctaaatt tgcaatatgt tcatgtaaac tatcgccttg tccagacact 1980

tgtgcttttt gaatgtcttc tttaaatgtc aaactatcat catggatcag ctgcataaaa 2040

ttgcgattgg caaaaccatc tgatttcaaa aaatctaata ttgttttgcc agattgctta 2100

tccctaatac cattaatcaa ttttcgagac aaacgtcccc aaccagtata acggcgacgt 2160

ttaagctgtt tcatcacctt atcatcaaag aggtgagcat atgttttaag tctttcctca 2220

atcatctccc tatcttcaaa taaggtcaat gttaaaacaa tatcctctaa gatatcttca 2280

ttttcttcat tatccaaaaa atctttatct ttaataattt ttagcaaatc atggtaggta 2340

cctaatgaag cattaaatct atcttcaact cctgaaattt caacactatc aaaacattct 2400

atttttttga aataatcttc ttttaattgc ttaacggtta cttttcgatt tgttttgaag 2460

agtaaatcaa caatggcttt cttctgttca cctgaaagaa atgctggttt tcgcattcct 2520

tcagtaacat atttgacctt tgtcaattcg ttataaaccg taaaatactc ataaagcaaa 2580

ctatgttttg gtagtacttt ttcatttgga agatttttat caaagtttgt catgcgttca 2640

ataaatgatt gagctgaagc acctttatcg acaacttctt caaaattcca tggggtaatt 2700

gtttcttcag acttccgagt catccatgca aaacgactat tgccacgcgc caatggacca 2760

acataataag gaattcgaaa agtcaagatt ttttcaatct tctcacgatt gtcttttaaa 2820

aatggataaa agtcttcttg tcttctcaaa atagcatgca gctcacccaa gtgaatttga 2880

tggggaatag agccgttgtc aaaggtccgt tgcttgcgca gcaaatcttc acgatttagt 2940

ttcaccaata attcctcagt accatccatt ttttctaaaa ttggtttgat aaatttataa 3000

aattcttctt ggctagctcc cccatcaata taacctgcat atccgttttt tgattgatca 3060

aaaaagattt ctttatactt ttctggaagt tgttgtcgaa ctaaagcttt taaaagagtc 3120

aagtcttgat gatgttcatc gtagcgttta atcattgaag ctgatagggg agccttagtt 3180

atttcagtat ttactcttag gatatctgaa agtaaaatag catctgataa attcttagct 3240

gccaaaaaca aatcagcata ttgatctcca atttgcgcca ataaattatc taaatcatca 3300

tcgtaagtat cttttgaaag ctgtaattta gcatcttctg ccaaatcaaa atttgattta 3360

aaattagggg tcaaacccaa tgacaaagca atgagattcc caaataagcc atttttcttc 3420

tcaccgggga gctgagcaat gagattttct aatcgtcttg atttactcaa tcgtgcagaa 3480

agaatcgctt tagcatctac tccacttgcg ttaatagggt tttcttcaaa taattgattg 3540

taggtttgta ccaactggat aaatagtttg tccacatcac tattatcagg atttaaatct 3600

ccctcaatca aaaaatgacc acgaaactta atcatatgcg ctaaggccaa atagattaag 3660

cgcaaatccg ctttatcagt agaatctacc aatttttttc gcagatgata gatagttgga 3720

tatttctcat gataagcaac ttcatctact atatttccaa aaataggatg acgttcatgc 3780

ttcttgtctt cttccaccaa aaaagactct tcaagtcgat gaaagaaact atcatctact 3840

ttcgccatct catttgaaaa aatctcctgt agataacaaa tacgattctt ccgacgtgta 3900

taccttctac gagctgtccg tttgagacga gtcgcttccg ctgtctctcc actgtcaaat 3960

aaaagagccc ctataagatt ttttttgata ctgtggcggt ctgtatttcc cagaaccttg 4020

aactttttag acggaacctt ataatcatca gtgatcaccg cccatccgac gctatttgtg 4080

ccgatatcta agcctattga gtatttctta tccattctaa ctaacctcct aaattttgat 4140

acggggtaac agataaacca tttcaatcta tttcataagt tccatagttt atccctaatt 4200

tatacgtttt ctctaacaac ttaattatac ccactattat tatttttatc aatatatttt 4260

gttaaaaagt cgactttata tttagtccct tgccttgcct acaaggattt tcctattcct 4320

ttcatttaca attcatacgt ataaaatcca aatttttctt gacatttata cacataaata 4380

ttatgattta tataggtaat cgctttcata aaatatatta cccttaggaa atcaaatgat 4440

tataagtcat atatgaaaac gttatatata attgatatgt ttacatttgt aacttagatt 4500

tctctttgat ttccacatat ataaatctta aggaggagtt ttcccaagca ttgataatga 4560

agggttttag agctagaaat agcaagttaa aataaggcta gtccgttatc aacttgaaaa 4620

agtggcaccg agtcggtgct ttttttataa aaataagaag cctgcatttg caggcttctt 4680

atttttatgc ggaatggaga tatcgtaata gctgatgctg cagaagatga aactgtcggg 4740

aaatgcagtg aaatcaaagg aataggttgt ataagtattg tctctggatt gcatacgata 4800

ccttgtagac ctatcaagac atttgagact ggttaccttg gatattatat gaattcaagc 4860

gcttatcatg atcagttatt accactgata cagggtacaa aaatttcatc tatttcaaag 4920

tcggcactac agaacacgga aataatttac ccagattcag aaaaggaaca actaaaaatt 4980

gggcagtttt ttcaaaacct ggatagcctt atcactcttc atcagcgaaa gtatgataag 5040

ctaattattg ttaaaaagtc tatgttggaa aaaatgttcc ctatagatgg tagcggtagc 5100

aatgtgcctg aaattcgatt tggtggtttt actgatgatt ggaaatttcg taagttggga 5160

gactgctttt cagaaaggtc tgaaagtatg cctgatgggg aattgatttc tgttactata 5220

aatgatggaa taaagaaatt ttcagaactt ggaagacatg atacttctaa cgatgataaa 5280

tctaagtata agaaggtgtg tgttggagac atagcctata actctatgag aatgtggcaa 5340

ggtgcaagtg gatactcccc atacgaaggt atagttagtc ctgcatatac agtacttgcc 5400

ccaaataatg gaattgactc aaaatgtatt tcatatttat ttaaacgccc tgatatgatt 5460

catacattcc aagttaattc acaaggaata acatctgata actggaattt gaaatatcag 5520

gcattgagtg aaattgagat tttgattcca aatgatattc aagaacaaaa atatattgct 5580

gaatacttca ctggtctcga caacctgatc actcttcatc agcgtaaact cgaaaaatta 5640

agaaatataa ggttttcttg tacggaaaaa atgtttgttt aggggggctg agattgtaat 5700

gtttagattt agaccagatt aacgtctggt ctattttttt gctcaaaaaa attttttgtt 5760

gaactttgtt caacgaaatc tctcactaat ttggagaatt tttaaatgca gaattaaata 5820

tgtgcgttat taaggctttt tggatgttga actttattca acgggcaaaa aagctagaaa 5880

gtataaaatg aatttatagt aaaaatcaat atcacaggcc tgattagcta taagggcatg 5940

ggatacaaat attggtccat cacagctaca gcgtgatggg tgcaatagaa gtacccttat 6000

ttccttatgc tatttttcag gcgatgggtc ggtgtacttc aaaaggcacc gactctattt 6060

gtttctcttg tccttctgca agaaccaggc agaaaggcag gaaactttat gagaaacttt 6120

aaaaccagca aaaagaacag aaccaattac atttattatt cagctgatgg acaggcaata 6180

aaaattaaac ctaatgagga tgaagctaca actactatca ttgtcacttt acatagctgg 6240

gatgatgcag aatttaatgc tgataaacgc gaaagatacc atgttccagt atacatggat 6300

gcttattgta atgaaaacga agatgttgca gctgatcgca atccatattt agttgatact 6360

gcttctgatc cactggaaag tattattcaa tccattgagg aaacagagca taaagaaaag 6420

ataggcaggc taagagctgc aattggaact ttaaagccac agcagaaaga gcttataaaa 6480

aaagtattct ttgaaaaacg cactaatgta agtattgctg atgaagaagg tgttacagaa 6540

gcagctatta gaaatcggct aaaaaagatt tatgagaaac ttcgcaaaaa aatctaaaaa 6600

taggggcacg aatcccccta aataaagttt caaaaaaggg ggttcgattc cctaaagttt 6660

tttgcatatg gacagagggg taaataaaac ccctcctcga g 6701

<210> 3
<211> 43
<212> DNA
<213> Artificial

<220>
<223> Synthetic DNA

<400> 3
gatccgcggc cgctctagag tcgacgggcc cctcgagcct cga 43

<210> 4
<211> 13255
<212> DNA
<213> Artificial

<220>

<223>  Vector pCL2-TypeIKO

<400>  4
gaattcgagc tcggtacccg gggatccgcg gccgctcagt cacctcctag ctgactcaaa      60

tcaatgcgtg tttcataaag accagtgatg gattgatgga taagagtggc atctaaaact     120

tcttttgtag acgtatatcg tttacgatca attgttgtat caaaatattt aaaagcagcg     180

ggagctccaa gattcgtcaa cgtaaataaa tgaataatat tttctgcttg ttcacgtatt     240

ggtttgtctc tatgtttgtt atatgcacta agaactttat ctaaattggc atctgctaaa     300

ataacacgct tagaaaattc actgatttgc tcaataatct catctaaata atgcttatgc     360

tgctccacaa acaattgttt ttgttcgtta tcttctggac tacccttcaa cttttcataa     420

tgactagcta aatataaaaa attcacatat ttgcttggca gagccagctc atttcctttt     480

tgtaattctc cggcactagc cagcatccgt ttacgaccgt tttctaactc aaaaagacta     540

tatttaggta gtttaatgat taagtctttt ttaacttcct tatatccttt agcttctaaa     600

aagtcaatcg gatttttttc aaaggaactt ctttccataa ttgtgatccc tagtaactct     660

ttaacggatt ttaacttctt cgatttccct ttttccacct tagcaaccac taggactgaa     720

taagctaccg ttggactatc aaaaccacca tatttttttg gatcccagtc ttttttacga     780

gcaataagct tgtccgaatt tctttttggt aaaattgact ccttggagaa tccgcctgtc     840

tgtacttctg ttttcttgac aatattgact tggggcatgg acaatacttt gcgcactgtg     900

gcaaaatctc gccctttatc ccagacaatt tctccagttt ccccattagt ttcgattaga     960

gggcgtttgc gaatctctcc atttgcaagt gtaatttctg ttttgaagaa gttcatgata    1020

ttagagtaaa agaaatattt tgcggttgct ttgcctattt cttgctcaga cttagcaatc    1080

attttacgaa catcataaac tttataatca ccatagacaa actccgattc aagttttgga    1140

tatttcttaa tcaaagcagt tccaacgacg gcatttagat acgcatcatg ggcatgatgg    1200

taattgttaa tctcacgtac tttatagaat tggaaatctt ttcggaagtc agaaactaat    1260

ttagatttta aggtaatcac tttaacctct cgaataagtt tatcattttc atcgtattta    1320

gtattcatgc gactatccaa aatttgtgcc acatgcttag tgatttggcg agtttcaacc    1380

aattggcgtt tgataaaacc agctttatca agttcactca aacctccacg ttcagctttc    1440

gttaaattat caaacttacg ttgagtgatt aacttggcgt ttagaagttg tctccaatag    1500

tttttcatct ttttgactac ttcttcactt ggaacgttat ccgatttacc acgattttta    1560

tcagaacgcg ttaagacctt attgtctatt gaatcgtctt taaggaaact ttgtggaaca    1620

atgtgatcga catcataatc acttaaacga ttaatatcta attcttggtc cacatacatg    1680

tctcttccat tttggagata atagagatag agcttttcat tttgcaattg agtattttca    1740

acaggatgct ctttaagaat ctgacttcct aattctttga taccttcttc gattcgtttc 1800

atacgctctc gcgaattttt ctggcccttt tgagttgtct gattttcacg tgccatttca 1860

ataacgatat tttctggctt atgccgcccc attactttga ccaattcatc aacaactttt 1920

acagtctgta aaataccttt tttaatagca gggctaccag ctaaatttgc aatatgttca 1980

tgtaaactat cgccttgtcc agacacttgt gctttttgaa tgtcttcttt aaatgtcaaa 2040

ctatcatcat ggatcagctg cataaaattg cgattggcaa aaccatctga tttcaaaaaa 2100

tctaatattg ttttgccaga ttgcttatcc ctaataccat taatcaattt tcgagacaaa 2160

cgtccccaac cagtataacg gcgacgttta agctgtttca tcaccttatc atcaaagagg 2220

tgagcatatg ttttaagtct ttcctcaatc atctccctat cttcaaataa ggtcaatgtt 2280

aaaacaatat cctctaagat atcttcattt tcttcattat ccaaaaaatc tttatcttta 2340

ataattttta gcaaatcatg gtaggtacct aatgaagcat taaatctatc ttcaactcct 2400

gaaatttcaa cactatcaaa acattctatt tttttgaaat aatcttcttt taattgctta 2460

acggttactt ttcgatttgt tttgaagagt aaatcaacaa tggctttctt ctgttcacct 2520

gaaagaaatg ctggttttcg cattccttca gtaacatatt tgacctttgt caattcgtta 2580

taaaccgtaa aatactcata aagcaaacta tgttttggta gtactttttc atttggaaga 2640

tttttatcaa agtttgtcat gcgttcaata aatgattgag ctgaagcacc tttatcgaca 2700

acttcttcaa aattccatgg ggtaattgtt tcttcagact tccgagtcat ccatgcaaaa 2760

cgactattgc cacgcgccaa tggaccaaca taataaggaa ttcgaaaagt caagattttt 2820

tcaatcttct cacgattgtc ttttaaaaat ggataaaagt cttcttgtct tctcaaaata 2880

gcatgcagct cacccaagtg aatttgatgg ggaatagagc cgttgtcaaa ggtccgttgc 2940

ttgcgcagca aatcttcacg atttagtttc accaataatt cctcagtacc atccattttt 3000

tctaaaattg gtttgataaa tttataaaat tcttcttggc tagctccccc atcaatataa 3060

cctgcatatc cgttttttga ttgatcaaaa aagatttctt tatacttttc tggaagttgt 3120

tgtcgaacta aagcttttaa aagagtcaag tcttgatgat gttcatcgta gcgtttaatc 3180

attgaagctg ataggggagc cttagttatt tcagtattta ctcttaggat atctgaaagt 3240

aaaatagcat ctgataaatt cttagctgcc aaaaacaaat cagcatattg atctccaatt 3300

tgcgccaata aattatctaa atcatcatcg taagtatctt ttgaaagctg taatttagca 3360

tcttctgcca aatcaaaatt tgatttaaaa ttaggggtca aacccaatga caaagcaatg 3420

agattcccaa ataagccatt tttcttctca ccggggagct gagcaatgag attttctaat 3480

cgtcttgatt tactcaatcg tgcagaaaga atcgctttag catctactcc acttgcgtta 3540

atagggtttt cttcaaataa ttgattgtag gtttgtacca actggataaa tagtttgtcc 3600

acatcactat tatcaggatt taaatctccc tcaatcaaaa aatgaccacg aaacttaatc 3660

atatgcgcta aggccaaata gattaagcgc aaatccgctt tatcagtaga atctaccaat 3720

ttttttcgca gatgatagat agttggatat ttctcatgat aagcaacttc atctactata 3780

tttccaaaaa taggatgacg ttcatgcttc ttgtcttctt ccaccaaaaa agactcttca 3840

agtcgatgaa agaaactatc atctactttc gccatctcat ttgaaaaaat ctcctgtaga 3900

taacaaatac gattcttccg acgtgtatac cttctacgag ctgtccgttt gagacgagtc 3960

gcttccgctg tctctccact gtcaaataaa agagccccta taagattttt tttgatactg 4020

tggcggtctg tatttcccag aaccttgaac tttttagacg gaaccttata atcatcagtg 4080

atcaccgccc atccgacgct atttgtgccg atatctaagc ctattgagta tttcttatcc 4140

attctaacta acctcctaaa ttttgatacg gggtaacaga taaaccattt caatctattt 4200

cataagttcc atagtttatc cctaatttat acgttttctc taacaactta attataccca 4260

ctattattat ttttatcaat atattttgtt aaaaagtcga ctttatattt agtcccttgc 4320

cttgcctaca agggatttcc tattcctttc atttacaatt catacgtata aaatccaaat 4380

ttttcttgac atttatacac ataaatatta tgatttatat aggtaatcgc tttcataaaa 4440

tatattaccc ttaggaaatc aaatgattat aagtcatata tgaaaacgtt atatataatt 4500

gatatgttta catttgtaac ttagatttct ctttgatttc cacatatata aatcttaagg 4560

aggagttttc ccaagcattg ataatgaagg gttttagagc tagaaatagc aagttaaaat 4620

aaggctagtc cgttatcaac ttgaaaaagt ggcaccgagt cggtgctttt tttataaaaa 4680

taagaagcct gcatttgcag gcttcttatt tttatgcgga atggagatat cgtaatagct 4740

gatgctgcag aagatgaaac tgtcgggaaa tgcagtgaaa tcaaaggaat aggttgtata 4800

agtattgtct ctggattgca tacgatacct tgtagaccta tcaagacatt tgagactggt 4860

taccttggat attatatgaa ttcaagcgct tatcatgatc agttattacc actgatacag 4920

ggtacaaaaa tttcatctat ttcaaagtcg gcactacaga acacggaaat aatttaccca 4980

gattcagaaa aggaacaact aaaaattggg cagttttttc aaaacctgga tagccttatc 5040

actcttcatc agcgaaagta tgataagcta attattgtta aaaagtctat gttggaaaaa 5100

atgttcccta tagatggtag cggtagcaat gtgcctgaaa ttcgatttgg tggttttact 5160

gatgattgga aatttcgtaa gttgggagac tgcttttcag aaaggtctga aagtatgcct 5220

gatggggaat tgatttctgt tactataaat gatggaataa agaaattttc agaacttgga 5280

agacatgata cttctaacga tgataaatct aagtataaga aggtgtgtgt tggagacata 5340

gcctataact ctatgagaat gtggcaaggt gcaagtggat actccccata cgaaggtata 5400

gttagtcctg catatacagt acttgcccca aataatggaa ttgactcaaa atgtatttca 5460
tatttattta aacgccctga tatgattcat acattccaag ttaattcaca aggaataaca 5520
tctgataact ggaatttgaa atatcaggca ttgagtgaaa ttgagatttt gattccaaat 5580
gatattcaag aacaaaaata tattgctgaa tacttcactg gtctcgacaa cctgatcact 5640
cttcatcagc gtaaactcga aaaattaaga aatataaggt tttcttgtac ggaaaaaatg 5700
tttgtttagg ggggctgaga ttgtaatgtt tagatttaga ccagattaac gtctggtcta 5760
tttttttgct caaaaaaatt ttttgttgaa ctttgttcaa cgaaatctct cactaatttg 5820
gagaattttt aaatgcagaa ttaaatatgt gcgttattaa ggctttttgg atgttgaact 5880
ttattcaacg ggcaaaaaag ctagaaagta taaaatgaat ttatagtaaa aatcaatatc 5940
acaggcctga ttagctataa gggcatggga tacaaatatt ggtccatcac agctacagcg 6000
tgatgggtgc aatagaagta cccttatttc cttatgctat ttttcaggcg atgggtcggt 6060
gtacttcaaa aggcaccgac tctatttgtt tctcttgtcc ttctgcaaga accaggcaga 6120
aaggcaggaa actttatgag aaactttaaa accagcaaaa agaacagaac caattacatt 6180
tattattcag ctgatggaca ggcaataaaa attaaaccta atgaggatga agctacaact 6240
actatcattg tcactttaca tagctgggat gatgcagaat ttaatgctga taaacgcgaa 6300
agataccatg ttccagtata catggatgct tattgtaatg aaaacgaaga tgttgcagct 6360
gatcgcaatc catatttagt tgatactgct tctgatccac tggaaagtat tattcaatcc 6420
attgaggaaa cagagcataa agaaaagata ggcaggctaa gagctgcaat tggaacttta 6480
aagccacagc agaaagagct tataaaaaaa gtattctttg aaaaacgcac taatgtaagt 6540
attgctgatg aagaaggtgt tacagaagca gctattagaa atcggctaaa aaagatttat 6600
gagaaacttc gcaaaaaaat ctaaaaatag gggcacgaat ccccctaaat aaagtttcaa 6660
aaaagggggt tcgattccct aaagtttttt gcatatggac agaggggtaa ataaaacccc 6720
tcctcgagcc ctgcaggcat gcttggcact ggccgtcgtt ttacaacgtc gtgactggga 6780
aaaccctggc gttacccaac ttaatcgcct tgcagcacat ccccctttcg ccagctggcg 6840
taatagcgaa gaggcccgca ccgatcgccc ttcccaacag ttgcgcagcc tgaatggcga 6900
atggcgcctg atgcggtatt ttctccttac gcatctgtgc ggtatttcac accgcatatg 6960
gtgcactctc agtacaatct gctctgatgc cgcatagtta agccagcccc gacacccgcc 7020
aacacccgct gacgcgccct gacgggcttg tctgctcccg gcatccgctt acagacaagc 7080
tgtgaccgtc tccgggagct gcatgtgtca gaggttttca ccgtcatcac cgaaacgcgc 7140
gagacgaaag ggcctcgtga tacgcctatt tttataggtt aatgtcatga taataatggt 7200
ttcttagacg tcaggtggca cttttcgggg aaatgtgcgc ggaaccccta tttgtttatt 7260

tttctaaata cattcaaata tgtatccgct catgagacaa taaccctgat aaatgcttca 7320

ataatattga aaaaggaaga gtatgagtat tcaacatttc cgtgtcgccc ttattccctt 7380

ttttgcggca ttttgccttc ctgtttttgc tcacccagaa acgctggtga aagtaaaaga 7440

tgctgaagat cagttgggtg cacgagtggg ttacatcgaa ctggatctca acagcggtaa 7500

gatccttgag agttttcgcc ccgaagaacg ttttccaatg atgagcactt ttaaattaaa 7560

aatgaagttt taaaacttca tttttaattt aaattaaaaa tgaagtttta tcaaaaaaat 7620

ttccaataat cccactctaa gccacaaaca cgccctataa aatcccgctt taatcccact 7680

ttgagacaca tgtaatatta ctttacgccc tagtatagtg ataatttttt acattcaatg 7740

ccacgcaaaa aaataaaggg gcactataat aaaagttcct tcggaactaa ctaaagtaaa 7800

aaattatctt tacaacctcc ccaaaaaaaa gaacaggtac aaagtaccct ataatacaag 7860

cgtaaaaaaa tgagggtaaa aataaaaaaa taaaaaaata aaaaaataaa aaaataaaaa 7920

aaataaaaaa ataaaaaaat aaaaaaataa aaaaataaaa aaataaaaaa ataaaaaaat 7980

aaaaaaatat aaaaataaaa aaatataaaa ataaaaaaat ataaaaataa aaaaatataa 8040

aaataaaaaa ataaaaaaat ataaaaataa aaaaataaaa aaatataaaa atatttttta 8100

tttaaagttt gaaaaaaatt tttttatatt atataatctt tgaagaaaag aatataaaaa 8160

atgagccttt ataaaagccc attttttttc atatacgtaa tatgacgttc taatgttttt 8220

attggtactt ctaacattag agtaatttct ttatttttaa agcctttttc tttaagggct 8280

tttatttttt ttcttaatac atttaattcc tctttttttg ttgcttttcc tttagctttt 8340

aattgctctt gataattttt tttacctcta atattttctc ttctcttata ttccttttta 8400

gaaattatta ttgtcatata tttttgttct tcttctgtaa tttctaataa ctctataaga 8460

gtttcattct tatacttata ttgcttattt ttatctaaat aacatctttc agcacttcta 8520

gttgctctta taacttctct ttcacttaaa tgttgtctaa acatactatt aagttctaaa 8580

acatcattta atgccttctc aatgtcttct gtaaagctac aaagataata tctatataaa 8640

aataatataa gctctctgtg tccttttaaa tcatattctc ttagttcaca aagttttatt 8700

atgtcttgta ttcttccata atataaactt ctttctctat aaatataatt tatttgcttt 8760

ggtctaccct ttttcctttc atatggtttt aattcaggta aaaatccatt ttgtatttct 8820

cttaagtcat aaatatattc gtactcatct aatatattga ctactgtttt tgatttagag 8880

tttatacttc ctggaactct taatattctg gttgcatcta aggcttgtct atctgctcca 8940

aagtatttta attgattata taaatattct tgaaccgctt tccataatgg taatgcttta 9000

ctaggtactg catttattat ccatattaaa tacattcctc ttccactatc tattacatag 9060

tttggtatag gaatactttg attaaaataa ttcttttcta agtccattaa tacctggtct 9120

ttagttttgc cagttttata ataatccaag tctataaaca gtgtatttaa ctcttttata 9180

ttttctaatc gcctacacgg cttataaaag gtatttagag ttatatagat attttcatca 9240

ctcatatcta aatcttttaa ttcagcgtat ttatagtgcc attggctata tcctttttta 9300

tctataacgc tcctggttat ccacccttta cttctactat gaatattatc tatatagttc 9360

tttttattca gctttaatgc gtttctcact tattcacctc cccttctgta aaactaagaa 9420

aattatatca tattttcaat aattattaac tattcttaaa ctcttaataa aaaatagagt 9480

aagtccccaa ttgaaactta atctattttt tatgttttaa tttattattt ttattaaaat 9540

attttaaact aaattaaatg attcttttta attttttact atttcattcc ataatatatt 9600

actataatta tttacaaata atatttcttc atttgtaata tttagatgat ttactaattt 9660

tagtttttat atattaaata attaatgtat aatttatata aaaaatcaaa ggagcttata 9720

aattatgatt atttccaaag atactaaaga tttaattttt tcaattttaa caatactttt 9780

tgtaatatta tgtttaaatt taattgtatt tttttcatat aataaagccg ttgaagtaaa 9840

ccaatccatt ttccttatga tgttattatt aaatttaagt tttataataa tatctttatt 9900

atatttattg tttttaaaaa aactagtgaa atttccggct ttattaaact tatttttagg 9960

aattttattt tcattttcat ctttacagga tttgattata tctttaaata tgttttatca 10020

aatattatct ttttctaaat ttatatatat ttttattata tttattatta tatatatttt 10080

atttttaagt ttctttctaa cagctattaa aaagaaactt aaaaataaaa acacgtactc 10140

taaaccaata aataaaacta tttttattat tgctgccttg attggaatag tttttagtaa 10200

aattaatttc aatattccac aatattatat tataagctag ctttgcattg tacttttcaa 10260

tcgcttcacg aatgcggtta tctccgaaag ataaagtctt ttcatcttcc ttgatgaaga 10320

taagattttc tccgtctccg ccggcagaat tgaagcgggg tactacggta tcgtctgcgt 10380

catcttccgt tgtctgatag atgatagtca taggctcatt ttcttccgtt tcggtaaagg 10440

ggataggttc gccctttgag agcagggcgg cgatggaaag cattaacttg cttttcccat 10500

cgcccggatc tccctgcaat agcgtaactt tgccaaacgg aatatacgga taccacagcc 10560

actttacttc tttcggctcg atttcacttg ccttgatgat ttcaagaggt acgctgaaat 10620

tcatttcgtt ttcatttagt ttcatttttt cttgttctcc ttttctctga aaatataaaa 10680

accacagatt gatactaaaa ccttggttgt gttgcttttc ggggcttaaa tcaaggaaaa 10740

atccttgttt taagcctttc aaaaagaaac acaaggtctt tgtactaacc tgtggttatg 10800

tataaaattg tagattttag ggtaacaaaa aacaccgtat ttctacgatg tttttgctta 10860

aatacttgtt tttagttaca gacaaacctg aagttaacta tttatcaatt cctgcaattc 10920

gtttacaaaa cggcaaatgt gaaatccgtc acatactgcg tgatgaactt gaattgccaa 10980

aggaagtata attttgttat cttctttata atatttcccc atagtaaaaa taggaatcaa 11040

ataatcatat cctttctgca aattcagatt aaagccatcg aaggttgacc acggtatcat 11100

agatacatta aaaatgtttt ccggagcatt tggctttcct tccattctat gattgtttcc 11160

ataccgttgc gtatcacttt cataatctgc taaaaatgat ttaaagtcag acttacactc 11220

agtccaaagg ctggaaaatg tttcagtatc attgtgaaat attgtatagc ttggtatcat 11280

ctcatcatat atccccaatt caccatcttg attgattgcc gtcctaaact ctgaatggcg 11340

gtttacaatc attgcaatat aataaagcat tgcaggatat agtttcattc cctttccctt 11400

tatttgtgtg atatccactt taacggtcat gctgtaagta caaggtacac ttgcaaagta 11460

gtggtcaaaa tactcttttc tgttccaact atttttatca attttttcaa ataccatcta 11520

agttccctct caaattcaag tttatcgctc taatgaacaa agatattata ccacattttt 11580

gtgaattttt caacttgccc acttcgactg cactcccgac ttaataactt cttgaacact 11640

tgccgaaaaa gaaaaactgc cgggtacgta cccgggatcg atccccgccg agcgcttagt 11700

gggaatttgt accccttatc gatacaaatt ccccgtaggc gctagggaca ctttttcact 11760

cgttaaaaag ttttgagaat attttatatt tttgttcatg taatcactcc ttcttaatta 11820

caaattttta gcatctaatt taacttcaat tcctattata caaaatttta agatactgca 11880

ctatcaacac actcttaagt ttgcttctaa gtcttatttc cataacttct tttacgtttc 11940

cgggtacaat tcgtaatcat gtcatagctg tttcctgtgt gaaattctta tccgctcaca 12000

attccacaca acatacgagc cggaagcata aagtgtaaag cctggggtgc ctaatgagtg 12060

agctaactca cattaattgc gttgcgctca ctgcccgctt tccagtcggg aaacctgtcg 12120

tgccagaaaa cttcattttt aatttaaaag gatctaggtg aagatccttt ttgataatct 12180

catgaccaaa atcccttaac gtgagttttc gttccactga gcgtcagacc ccgtagaaaa 12240

gatcaaagga tcttcttgag atcctttttt tctgcgcgta atctgctgct tgcaaacaaa 12300

aaaaccaccg ctaccagcgg tggtttgttt gccggatcaa gagctaccaa ctctttttcc 12360

gaaggtaact ggcttcagca gagcgcagat accaaatact gtccttctag tgtagccgta 12420

gttaggccac cacttcaaga actctgtagc accgcctaca tacctcgctc tgctaatcct 12480

gttaccagtg gctgctgcca gtggcgataa gtcgtgtctt accgggttgg actcaagacg 12540

atagttaccg gataaggcgc agcggtcggg ctgaacgggg ggttcgtgca cacagcccag 12600

cttggagcga acgacctaca ccgaactgag atacctacag cgtgagctat gagaaagcgc 12660

cacgcttccc gaagggagaa aggcggacag gtatccggta agcggcaggg tcggaacagg 12720

agagcgcacg agggagcttc cagggggaaa cgcctggtat ctttatagtc ctgtcgggtt 12780

tcgccacctc tgacttgagc gtcgattttt gtgatgctcg tcaggggggc ggagcctatg 12840

gaaaaacgcc agcaacgcgg cctttttacg gttcctggcc ttttgctggc cttttgctca 12900

catgttcttt cctgcgttat cccctgattc tgtggataac cgtattaccg cctttgagtg 12960

agctgatacc gctcgccgca gccgaacgac cgagcgcagc gagtcagtga gcgaggaagc 13020

ggaagagcgc ccaatacgca aaccgcctct ccccgcgcgt tggccgattc attaatgcag 13080

ctggcacgac aggtttcccg actggaaagc gggcagtgag cgcaacgcaa ttaatgtgag 13140

ttagctcact cattaggcac cccaggcttt acactttatg cttccggctc gtatgttgtg 13200

tggaattgtg agcggataac aatttcacac aggaaacagc tatgaccatg attac 13255

<210> 5
<211> 6038
<212> DNA
<213> Artificial

<220>
<223> Vector pUC-Δpta-ermC

<400> 5
gacgaaaggg cctcgtgata cgcctatttt tataggttaa tgtcatgata ataatggttt 60

cttagacgtc aggtggcact tttcggggaa atgtgcgcgg aacccctatt tgtttatttt 120

tctaaataca ttcaaatatg tatccgctca tgagacaata accctgataa atgcttcaat 180

aatattgaaa aaggaagagt atgagtattc aacatttccg tgtcgccctt attccctttt 240

ttgcggcatt ttgccttcct gtttttgctc acccagaaac gctggtgaaa gtaaaagatg 300

ctgaagatca gttgggtgca cgagtgggtt acatcgaact ggatctcaac agcggtaaga 360

tccttgagag ttttcgcccc gaagaacgtt ttccaatgat gagcactttt aaagttctgc 420

tatgtggcgc ggtattatcc cgtattgacg ccgggcaaga gcaactcggt cgccgcatac 480

actattctca gaatgacttg gttgagtact caccagtcac agaaaagcat cttacggatg 540

gcatgacagt aagagaatta tgcagtgctg ccataaccat gagtgataac actgcggcca 600

acttacttct gacaacgatc ggaggaccga aggagctaac cgcttttttg cacaacatgg 660

gggatcatgt aactcgcctt gatcgttggg aaccggagct gaatgaagcc ataccaaacg 720

acgagcgtga caccacgatg cctgtagcaa tggcaacaac gttgcgcaaa ctattaactg 780

gcgaactact tactctagct tcccggcaac aattaataga ctggatggag gcggataaag 840

ttgcaggacc acttctgcgc tcggcccttc cggctggctg gtttattgct gataaatctg 900

gagccggtga gcgtgggtct cgcggtatca ttgcagcact ggggccagat ggtaagccct 960

cccgtatcgt agttatctac acgacgggga gtcaggcaac tatggatgaa cgaaatagac 1020

agatcgctga gataggtgcc tcactgatta agcattggta actgtcagac caagtttact 1080

catatatact ttagattgat ttaaaacttc atttttaatt taaaaggatc taggtgaaga 1140

tcctttttga taatctcatg accaaaatcc cttaacgtga gttttcgttc cactgagcgt 1200

cagaccccgt agaaaagatc aaaggatctt cttgagatcc tttttttctg cgcgtaatct 1260

gctgcttgca aacaaaaaaa ccaccgctac cagcggtggt ttgtttgccg gatcaagagc 1320

taccaactct ttttccgaag gtaactggct tcagcagagc gcagatacca aatactgttc 1380

ttctagtgta gccgtagtta ggccaccact tcaagaactc tgtagcaccg cctacatacc 1440

tcgctctgct aatcctgtta ccagtggctg ctgccagtgg cgataagtcg tgtcttaccg 1500

ggttggactc aagacgatag ttaccggata aggcgcagcg gtcgggctga acggggggtt 1560

cgtgcacaca gcccagcttg gagcgaacga cctacaccga actgagatac ctacagcgtg 1620

agctttgaga aagcgccacg cttcccgaag ggagaaaggc ggacaggtat ccggtaagcg 1680

gcagggtcgg aacaggagag cgcacgaggg agcttccagg gggaaacgcc tggtatcttt 1740

atagtcctgt cgggtttcgc cacctctgac ttgagcgtcg atttttgtga tgctcgtcag 1800

gggggcggag cctatggaaa aacgccagca acgcggcctt tttacggttc ctggcctttt 1860

gctggccttt tgctcacatg ttctttcctg cgttatcccc tgattctgtg gataaccgta 1920

ttaccgcctt tgagtgagct gataccgctc gccgcagccg aacgaccgag cgcagcgagt 1980

cagtgagcga ggaagcggaa gagcgcccaa tacgcaaacc gcctctcccc gcgcgttggc 2040

cgattcatta atgcagctgg cacgacaggt ttcccgactg gaaagcgggc agtgagcgca 2100

acgcaattaa tgtgagttag ctcactcatt aggcacccca ggctttacac tttatgcttc 2160

cggctcgtat gttgtgtgga attgtgagcg gataacaatt tcacacagga aacagctatg 2220

accatgatta cgaattccgt gcagaaggat aggacatacc ctgtgacatt ttttccttta 2280

aaaataattt aaattgggta ggctcttctg caagaatttt tgcaatagat ttcagcaagt 2340

ttatattact atattcgctt ccaaaacaaa gattttttac tacacccaag ttttctaaga 2400

gacttacagc accataggca aaaaattcag cagaagatag actgtagata acaggaagtt 2460

caaataccag gtctactcca tttagaagtg ccattttggt tttagtccat ttgtcaacta 2520

tagatggtga acctctttgc acgaagttac cactcataac tgctattaca gcatcacatt 2580

ttgtagcaga acgagcactt tcaatatgat atttatgtcc attgtgaaag ggattatatt 2640

caactattat tccagttacg ttcatagaaa ttttcctttc taaaatattt tattccatgt 2700

caagaactct gtttatttca ttaaagaact ataagtacaa agtatagggc atttgaaaaa 2760

ataggctagt atattgattg attatttatt ttaaaatgcc taagtgaaat atatacatat 2820

tataacaata aaataagtat tagtgtagga tttttaaata gagtatctat tttcagatta 2880

aatttttgct tatttgattt acattatata atattgagta aagtattgac tagcaaaatt 2940

ttttgatact ttaatttgtg aaatttctta tcaaaagtta tatttttgaa tgatttttat 3000

tgaaaaatac aactaaaaag gattatagta taagtgtgtg taattttgtg ttaaatttaa 3060

agggaggaaa tgaacatgaa attgatggaa aaaatttgga gtaaggcaaa ggaagacaaa 3120

aaaaagattg tcttagctga aggagaagaa gaaagaactc ttcaagcttg tgaaaaaata 3180

attaaagagg gtattgcaaa tttaatcctt gtagggaatg aaaaggtaat aaaagaaaaa 3240

gcgtcaaaat taggtgtaag tttaaatgga gcagaaatag tagatccaga gacttcagat 3300

aaactaaagg catatgcaga tgctttttat gaattgagaa agaagaaggg aataacgcca 3360

gaaaaagcgg atcttcggaa taggaacttc cgaccaaaag tataaaacct ttaagaactt 3420

tcttttttct tgtaaaaaaa gaaactagat aaatctctca tatcttttat tcaataatcg 3480

catcagattg cagtataaat ttaacgatca ctcatcatgt tcatatttat cagagctcgt 3540

gctataatta tactaatttt ataaggagga aaaaataaag agggttataa tgaacgagaa 3600

aaatataaaa cacagtcaaa actttattac ttcaaaacat aatatagata aaataatgac 3660

aaatataaga ttaaatgaac atgataatat ctttgaaatc ggctcaggaa aagggcattt 3720

tacccttgaa ttagtacaga ggtgtaattt cgtaactgcc attgaaatag accataaatt 3780

atgcaaaact acagaaaata aacttgttga tcacgataat ttccaagttt taaacaagga 3840

tatattgcag tttaaatttc ctaaaaacca atcctataaa atatttggta atatacctta 3900

taacataagt acggatataa tacgcaaaat tgtttttgat agtatagctg atgagattta 3960

tttaatcgtg gaatacgggt ttgctaaaag attattaaat acaaaacgct cattggcatt 4020

atttttaatg gcagaagttg atatttctat attaagtatg gttccaagag aatattttca 4080

tcctaaacct aaagtgaata gctcacttat cagattaaat agaaaaaaat caagaatatc 4140

acacaaagat aaacagaagt ataattattt cgttatgaaa tgggttaaca aagaatacaa 4200

gaaaatattt acaaaaaatc aatttaacaa ttccttaaaa catgcaggaa ttgacgattt 4260

aaacaatatt agctttgaac aattcttatc tcttttcaat agctataaat tatttaataa 4320

gtaagttaag ggatgcataa actgcatccc ttaacttgtt tttcgtgtac ctattttttg 4380

tgaatcgatt atgtcttttg cgcattcact tcttttctat ataaatatga gcgaagcgaa 4440

taagcgtcgg aaaagcagca aaaagtttcc tttttgctgt tggagcatgg gggttcaggg 4500

ggtgcagtat cgaagttcct ggaatggatc caaaagtagc aatgctttca ttttctacta 4560

agggaagtgc aaaacacgaa ttagtagaca aagttagaaa tgctgtagag attgcaaaaa 4620

aagctaaacc agatttaagt ttagacggag aattacaatt agatgcctct atcgtagaaa 4680

aggttgcaag tttaaaggct cctggaagtg aagtagcagg aaaagcaaat gtacttgtat 4740

ttccagatct ccaagcagga aatataggct ataaactcgt tcaaagattt gcaaaagcag 4800

atgctatagg acctgtatgc caaggatttg caaaacctat aaatgatttg tcaagaggat 4860

gtaattctga tgatatagta aatgtagtag ctgtaacagc agttcaagca caagctcaaa 4920

agtaataaca aaaagcataa atgattcatt tttaggagga atattaaaca tgaaaatatt 4980

agtagtaaac tgtggaagtt catctttaaa atatcaactt attgatatgc aagatgaaag 5040

tgttgtagca aagggtcttg tagaaagaat aggaatggac ggttcaattt taacacacaa 5100

agttaatgga gaaaagtttg ttacagagca aacaatggaa gaccacaaag ttgctataca 5160

attagtatta aatgctcttg tagataaaaa acatggtgta ataaaagaca tgtcagaaat 5220

atccgctgta ggacatagag tcttgcacgg tggaaagaaa tatgcagcat ccattcttat 5280

tgacgaaaat gtaatgaaag caatagaaga atgtatccca ctaggaccac tacataatcc 5340

agctaatata atgggaatag atgcttgtaa aaaattaatg ccaaatactc caatggtagc 5400

agtatttgat acagcatttc atcagacaat gccagattat gcttatactt atgcaatacc 5460

ttatgatata tctgaaaagt atgatatcag aaaatatggt tttcatggaa cttctcatag 5520

attcgtttca attgaagcag ctaaattatt aaagaaagat ccaaaagatc ttaagttaat 5580

aacttgtcat ttaggaaatg gagctagcat atgtgcgtcg acctgcaggc atgcaagctt 5640

ggcactggcc gtcgttttac aacgtcgtga ctgggaaaac cctggcgtta cccaacttaa 5700

tcgccttgca gcacatcccc ctttcgccag ctggcgtaat agcgaagagg cccgcaccga 5760

tcgcccttcc caacagttgc gcagcctgaa tggcgaatgg cgcctgatgc ggtattttct 5820

ccttacgcat ctgtgcggta tttcacaccg catatggtgc actctcagta caatctgctc 5880

tgatgccgca tagttaagcc agccccgaca cccgccaaca cccgctgacg cgccctgacg 5940

ggcttgtctg ctcccggcat ccgcttacag acaagctgtg accgtctccg ggagctgcat 6000

gtgtcagagg ttttcaccgt catcaccgaa acgcgcga 6038

## Description

### TECHNICAL FIELD

The present invention relates to an obligately anaerobic acetogen, and a recombinant microorganism. The obligately anaerobic acetogen of the present invention has high transformation efficiency by a foreign gene.

### BACKGROUND ART

Obligately anaerobic acetogens have conventionally been used for fermentation production of butanol, acetone, and the like. In particular, a *Clostridium* bacterium is used for production of bioethanol and production of organic acid such as acetic acid and lactic acid, using a lignocellulose-based biomass or syngas (synthesis gas). A *Clostridium* bacterium is expected to be used in the field of medicine.

Recombinant microorganisms using obligately anaerobic acetogen such as a *Clostridium* bacterium as a host are useful to improve the production ability of a useful substance. However, in general, since the transformation efficiency of a *Clostridium* bacterium is very low, it is difficult to obtain desired recombinant microorganisms. This is a major impediment to development in the art.

In general, microorganisms have developed a restriction modification system to protect themselves from foreign DNA such as viruses and phages. The restriction modification system includes a restriction enzyme that cleaves DNA and a modification enzyme that methylates DNA. By the action of the modification enzyme, DNA that has been modified by methylation is protected from cleavage by restriction enzymes, and genomic DNA of the microorganism itself is not cleaved. The microorganism distinguishes the DNA of its own from a foreign DNA such as viruses, by the methylation modification, and protects itself by cleavage of the foreign DNA by restriction enzymes.

The restriction modification systems are roughly classified into four types, that is, type I, type II, type III, and type VI depending on a structure of subunit, a cleavage site, and characteristics of a cofactor. Restriction enzymes of type I, type II and type III restriction modification systems distinguish and cleave DNA that has not been methylated (see, for example, Non-Patent Document 1). However, when DNA is recognized and modified by a methylase that is a pair of the restriction enzyme, the restriction enzyme cannot cleave the DNA. On the other hand, the type IV restriction modification system includes only a restriction enzyme, and is a methylation-dependent restriction enzyme that distinguishes and cleaves DNA having methylation form of a foreign DNA. In this way, the restriction modification system is a strong system that protects microorganism cells from foreign DNA. Therefore, when foreign DNA is introduced into a microorganism to form a recombinant, it is important to overcome the restriction modification system of a host.

In bacteria, improvement of transformation efficiency using a restriction modification system, in particular, a type II restriction modification enzyme (methyltransferase) has been studied, and described in, for example, Patent Document 1. However, its implementation includes complex operations, for example, it needs a plurality of steps, and needs all methyltransferases of a host, so that it has many problems to be solved.

Furthermore, there has been no report that transformation efficiency is improved by modification (alteration) of the type I restriction modification system, in particular, by deletion of the type I restriction enzyme.

Incidentally, among the *Clostridium* bacteria, in particular, *Clostridium ljungdahlii* is much expected. *Clostridium ljungdahlii* has advantages in that the genome information is publicly available, culture is easy, and synthesis gas (syngas) can be assimilated.

The syngas is a mixed gas mainly including carbon monoxide, carbon dioxide and hydrogen, which is efficiently obtained from waste, natural gas and coal by the action of a metal catalyst at high temperature and pressure. The syngas including carbon monoxide, carbon dioxide, and hydrogen is produced and available almost permanently as syngas derived from waste or industrial waste gas, natural gas, or syngas derived from coal. Thus, a permanent production method of a substance using microorganisms having syngas assimilating ability has been expected. By using the recombinant microorganism, the productivity of a target substance can be expected to be improved. However, there are very few reports as to the use of the syngas assimilating microorganism as a recombinant. This is thought to be mainly because as mentioned above, the transformation efficiency in a *Clostridium* bacterium is low, so that the desired recombinant cannot be easily obtained.

### PRIOR ART DOCUMENTS

### PATENT DOCUMENTS

Patent Document 1: JP 2015-515268 A

### NON-PATENT DOCUMENTS

Non-Patent Document 1: Wil A. M. Loenen et al., Nucleic Acids Research, 2014, vol. 42(1), p.22-44

### DISCLOSURE OF INVENTION

### TECHNICAL PROBLEM

In view of the above-mentioned circumstances, the present invention has an object to provide an obligately anaerobic acetogen, such as a *Clostridium* bacterium whose transformation efficiency is improved.

### SOLUTION TO PROBLEM

One aspect of the present invention to solve the above-mentioned problem is a modified obligately anaerobic acetogen in which activity of at least one enzyme selected from the group consisting of a restriction enzyme, a modification enzyme, and a recognition enzyme constituting a type I restriction modification system enzyme is deleted or suppressed.

This aspect relates to a modified obligately anaerobic acetogen. In the obligately anaerobic acetogen of this aspect, the activity of at least one enzyme selected from the group consisting of a restriction enzyme, a modification enzyme, and a recognition enzyme constituting a type I restriction modification system enzyme is deleted or suppressed. Therefore, the function of the type I restriction modification system is lost or reduced. Since the obligately anaerobic acetogen of this aspect is tolerant of degradation (cleavage property) to foreign DNA, when it is used as a host, the transformation efficiency is high. When the obligately anaerobic acetogen of this aspect is used, a recombinant of the obligately anaerobic acetogen can be obtained at high efficiency.

Preferably, the activity of at least the restriction enzyme is deleted or suppressed.

Preferably, a part or whole of a gene encoding at least one enzyme selected from the group consisting of the restriction enzyme, the modification enzyme, and the recognition enzyme is deleted.

Preferably, a part or whole of a gene encoding at least the restriction enzyme is deleted.

Preferably, the activity of the restriction enzyme, the modification enzyme, or the recognition enzyme is deleted

Preferably, the obligately anaerobic acetogen is capable of proliferating using carbon monoxide or carbon dioxide as a sole carbon source.

Preferably, the obligately anaerobic acetogen includes carbon monoxide dehydrogenase.

Preferably, the obligately anaerobic acetogen has a function of synthesizing acetyl-CoA from methyl tetrahydrofolate, carbon monoxide, and CoA.

Preferably, the obligately anaerobic acetogen is a bacterium.

Preferably, the obligately anaerobic acetogen is a *Clostridium* bacterium or a *Moorella* bacterium.

Preferably, the obligately anaerobic acetogen is *Clostridium ljungdahlii*.

Preferably, the restriction enzyme is a protein including an amino acid sequence of SEQ ID NO: 1.

Preferably, the obligately anaerobic acetogen is *Clostridium ljungdahlii*, the activity of at least the restriction enzyme is deleted or suppressed, and a part or whole of a gene encoding at least the restriction enzyme is deleted.

Another aspect of the present invention is a recombinant microorganism including the above-described obligately anaerobic acetogen into which a foreign gene encoding a target protein is introduced, and being capable of expressing the target protein.

This aspect relates to a recombinant microorganism. In the recombinant microorganism of this aspect, a foreign gene encoding a target protein is introduced into the above-mentioned obligately anaerobic acetogen as a host, and the target protein can be expressed. Production of the recombinant microorganism of this aspect is easy.

Preferably, the foreign gene is incorporated into a genome.

### EFFECT OF INVENTION

With the obligately anaerobic acetogen of the present invention, a recombinant of an obligately anaerobic acetogen can be obtained with high efficiency.

The recombinant microorganism of the present invention can be easily produced.

### BRIEF DESCRIPTION OF DRAWINGS

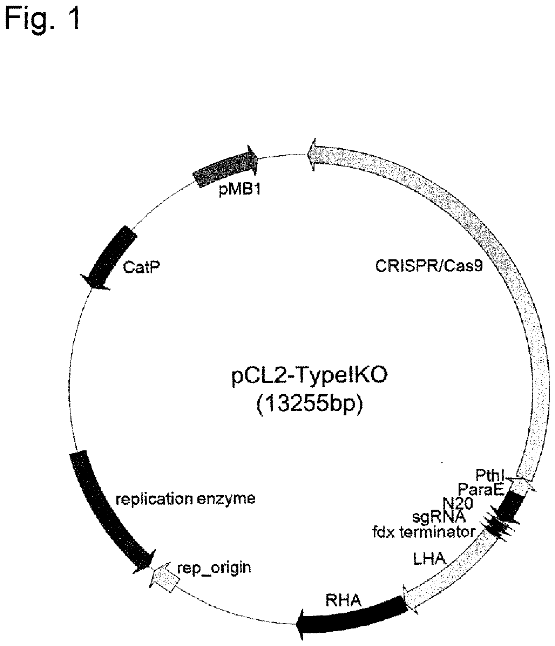

Fig. 1 is an explanatory diagram showing a configuration of a pCL2-TypeIKO vector.

### BEST MODE FOR CARRYING OUT THE INVENTION

Hereinafter, the exemplary embodiment of the present invention will be described. Note here that in the present invention, all the terms "gene" can be replaced with terms "nucleic acid" or "DNA".

In the modified obligately anaerobic acetogen of the present invention, activity of at least one enzyme selected from the group consisting of a restriction enzyme, a modification enzyme, and a recognition enzyme constituting a type I restriction modification system enzyme is deleted or suppressed. Herein, obligatory anaerobic property means a property which makes growth in the presence of oxygen virtually impossible and is the same meaning as strictly anaerobic property. Acetogen means a microorganism that produces acetate by anaerobic respiration.

In general, the type I restriction modification system enzyme functions as a complex consisting of three enzyme subunits, recognition, modification, and restriction (cleavage). The recognition enzyme (type I recognition enzyme) recognizes a DNA sequence that is specific to a species or specific to each enzyme. The modification enzyme (type I modification enzyme) carries out methylation by methyltransferase activity. The restriction enzyme (type I restriction enzyme) carries out cleavage of DNA by endonuclease activity.

It is known that when activity of at least one of these three enzymes is lost, the type I restriction modification system does not function (see, Non-Patent Document 1 mentioned above). In the obligately anaerobic acetogen of the present invention, activity of at least one enzyme selected from the group consisting of a restriction enzyme, a modification enzyme, and a recognition enzyme constituting the type I restriction modification system enzyme is deleted or suppressed. In other words, the function of the type I restriction modification system is lost or reduced.

Deletion of the enzyme activity means that activity of the enzyme is absent. Suppression of the enzyme activity means that as compared with the enzyme activity of a microorganism before modification or alteration (i.e. wild type), the activity is reduced to 50% or less, preferably 25% or less, and more preferably 10% or less. Measurement of the activity of the type I restriction modification system enzyme can be carried out by, for example, a method (DNA cleavage assay) described in Piero R. Bianco et al., Nucleic Acids Research, 2009, Vol. 37, No. 10, 3377-3390.

Examples of techniques for deleting or suppressing the activity of enzyme include roughly two techniques, a technique of reducing (including reducing to zero) the number of enzyme molecules to be expressed and a technique of reducing (including reducing to zero) the activity of the enzyme itself to be expressed.

Examples of the technique of reducing the number of enzyme molecules to be expressed include reducing the expression amount of the gene of the type I restriction modification system enzyme at a transcription level or a translation level. Specific examples of the technique include a method of modifying (altering) an expression regulatory sequence such as a promoter sequence and a Shine-Dalgarno (SD) sequence of the gene of the type I restriction modification system enzyme to reduce the expression amount of the gene.

Examples of the technique of reducing the activity of enzyme itself to be expressed include deleting a part or whole of the gene of the type I restriction modification system enzyme on a genome. Examples thereof include an embodiment in which some or all of the genes encoding the restriction enzyme, the modification enzyme, or the recognition enzyme are deleted. Among them, preferred is an embodiment in which some or all of the genes encoding at least a restriction enzyme are deleted.

Other techniques for reducing the activity of the enzyme itself to be expressed include introduction of mutation into a gene of the type I restriction modification system enzyme on the genome. For example, an objective microorganism is subjected to treatment with a mutagenic agent and the like so as to introduce mutation into a gene of the target type I restriction modification system enzyme. For example, objective microorganisms are treated by radiation irradiation or with mutagenic agents such as N-methyl-N'-nitro-N-nitroso guanidine (NTG) and nitrous acid, and then a mutant microorganism (modified microorganism) in which the activity of the type I restriction modification system enzyme is deleted or suppressed can be obtained.

The enzyme gene originally possessed by the host microorganism may be substituted with another gene having lower enzyme activity. The activity of the type I restriction modification system enzyme itself can be reduced by replacing, for example, an existing gene of the type I restriction modification system enzyme on the genome with a mutant gene that caused a frame shift in the gene, a mutant gene in which a point mutation is introduced in the gene, and a partial fragment gene of the gene.

Substitution of a gene can be carried out by a genome editing technique (CRISPR-Cas9 system). In the genome editing technique, any DNA sequences can be targeted site-specifically under the conditions in which Cas9 as endonuclease or mutant Cas9 that does not have catalyst activity form a complex with sgRNA (single guide RNA), in a vicinity of a region in which sgRNA and the target DNA are complementary bonded, and a specific short nucleotide sequence called PAM (proto-spacer adjacent motif) exists. Use of the genome editing technique in the syngas assimilating microorganism is described in, for example, "Huang H et al., ACS Synth Biol. 2016 Jun 15."

The substitution of the gene can also be carried out by homologous recombination. The sequence identity of nucleotide sequence required for the homologous recombination is preferably 70% or more, more preferably 80% or more, further preferably 90% or more, and particularly preferably 95% or more. Note here that gene manipulation methods by the homologous recombination have been established for many bacteria and the like, and the methods include a method using a linear DNA, and a method using a plasmid (see US 6303383 B1, JP H05-007491 A, etc.). Technique of homologous recombination by plasmid in a *Clostridium* bacterium is described in, for example, "Heap JT et al., J. Microbiol. Methods, 2007, vol.70 (3), p.452-64".

Together with a gene encoding Cas9 or a gene encoding sgRNA, a homologous DNA sequence for deleting a part or whole of the type I restriction modification enzyme may be introduced.

Some or all of the type I restriction modification system enzyme genes can be deleted by introducing a DNA sequence (DNA fragment) such as a foreign gene into an unmodified microorganism. For example, some or all of the type I restriction modification system enzyme gene can be deleted by inserting a DNA sequence into the structural gene of the type I restriction modification system enzyme. The DNA sequence to be introduced at this time is not particularly limited, and may be a DNA sequence originally possessed by the microorganism, or may be a heterologous DNA sequence that is not originally possessed by the microorganism. Examples of the DNA sequence to be introduced include an antibiotics resistant gene.

A method for introducing a DNA fragment into a microorganism is not particularly limited, and it may be appropriately selected depending on types of microorganisms. For example, it is possible to use a vector that can be introduced into a microorganism and that cannot autonomously replicate after recombination. For example, when a microorganism is a prokaryote such as a bacterium, it is possible to use a vector that can be incorporated into chromosome (genome) in a microorganism. For example, preferably, the above-mentioned DNA fragment is introduced using a vector, and then the vector is eliminated.

Examples of the type I restriction modification system enzyme of the obligately anaerobic acetogen include the followings.

Examples of type I restriction enzyme (for example, EC: 3.1.21.3):
ADK13415 (*Clostridium ljungdahlii*-hsdR); AKN29959 (*Clostridium carboxidivorans-hsdR*); AFA47678 (*Acetobacterium woodii*-hsdR1); ADO36134 (*Eubacterium limosum*-hsdR), and the like (all of which are shown as NCBI-ProteinID).

Note here that the type I restriction enzyme is also called a type I restriction enzyme R subunit, or a type I restriction enzyme endonuclease subunit.

Examples of type I recognition enzyme (for example, EC: 3.1.21.3):
ADK13414 (*Clostridium ljungdahlii*-hsdS); AKN31535 (*Clostridium carboxidivorans*-hsdS); AFA47246 (*Acetobacterium woodii*-hsdS1); ADO36135 (*Eubacterium limosum*-hsdS), and the like (all of which are shown as NCBI-ProteinID).

Note here that the type I recognition enzyme is also called a type I restriction enzyme S subunit (type I restriction enzyme, specificity subunit).

Examples of type I modification enzyme (for example, EC: 2.1.1.72):
ADK13413 (*Clostridium ljungdahlii*-hsdM); AKN33779 (*Clostridium carboxidivorans-hsdM*); AFA47243 (*Acetobacterium woodii*-hsdM1); ADO36136 (*Eubacterium limosum*-hsdM), and the like (all of which are shown as NCBI-ProteinID).

Note here that the type I modification enzyme is also called a type I modification enzyme M subunit (type I restriction enzyme M protein).

In the present invention, enzymes whose enzyme activity is deleted or suppressed may be any one or two or more of the restriction enzyme, modification enzyme, and recognition enzyme. Among them, preferably, the activity of at least the restriction enzyme is deleted or suppressed. Furthermore, when the obligately anaerobic acetogen includes a plurality of type I restriction modification system enzymes, the activity of at least one type I restriction modification system enzyme is only required to be deleted or suppressed. In addition, when a plurality of restriction enzymes, modification enzymes, or recognition enzymes are included, the activity of at least one of these is only required to be deleted or suppressed. For example, if a plurality of restriction enzymes are included, the activity of one of the restriction enzymes may be deleted or suppressed, or the activity of two or more, for example, all of the restriction enzymes may be deleted or suppressed.

As a specific example, the amino acid sequence of the type I restriction enzyme ADK13414 (hsdS) derived from *Clostridium ljungdahlii* is shown in SEQ ID NO: 1.

Preferably, the obligately anaerobic acetogen of the present invention can proliferate using carbon monoxide or carbon dioxide as a sole carbon source.

Furthermore, preferably, the obligately anaerobic acetogen of the present invention includes a carbon monoxide dehydrogenase. In detail, preferred is a microorganism that grows by the function of generating carbon dioxide and proton from carbon monoxide and water, mainly by carbon monoxide metabolism, that is, the action of the carbon monoxide dehydrogenase.

Furthermore, preferably, the obligately anaerobic acetogen of the present invention has a function of synthesizing acetyl-CoA from methyl tetrahydrofolate, carbon monoxide, and CoA. A pathway of synthesizing acetyl-CoA from methyl tetrahydrofolate, carbon monoxide, and CoA is included in, for example, a reduced acetyl-CoA pathway (Wood-Ljungdahl pathway) and Methanol pathway.

The obligately anaerobic acetogen of the present invention is preferably a microorganism having an acetyl-CoA pathway and further having carbon monoxide resistance and syngas assimilation property.

The obligately anaerobic acetogen of the present invention is preferably a bacterium, for example, a *Clostridium* bacterium or a *Moorella* bacterium, and particularly preferably *Clostridium ljungdahlii*.

Specific examples of microorganisms that can be employed as the obligately anaerobic acetogen of the present invention include *Clostridium* bacteria, *Moorella* bacteria, and *Acetobacterium* bacteria such as *Clostridium ljungdahlii*, *Clostridium carboxidivorans*, *Moorella thermoacetica* (the same as *Clostridium thermoaceticum*) (Pierce EG. Et al., Environ. Microbiol., 2008, vol.10, p.2550-2573), and *Acetobacterium woodii* (Dilling S. et al., Appl. Environ. Microbiol., 2007, vol.73 (11), p.3630-3636). These four anaerobic microorganisms are known as representative examples of the syngas assimilating microorganism. In particular, in the *Clostridium* bacterium, a host-vector system or a culture method has been established, and thus *Clostridium* bacterium is suitable as the obligately anaerobic acetogen of the present invention.

The other examples include *Carboxydocella sporoducens* sp. Nov. (Slepova TV. et al., Inter. J. Sys. Evol. Microbiol., 2006, vol.56, p.797-800), *Rhodopseudomonas gelatinosa* (Uffen RL, J. Bacteriol., 1983, vol. 155(3), p.956-965), *Eubacterium limosum* (Roh H. et al., J. Bacteriol., 2011, vol.193 (1), p.307-308), Butyribacterium methylotrophicum (Lynd, LH. Et al., J. Bacteriol., 1983, vol.153 (3), p.1415-1423).

Note here that all of proliferation and CODH activity of the bacteria mentioned above are oxygen sensitive. However, oxygen insensitive CODH is also known. For example, oxygen insensitive CODH exists in other bacterial species represented by *Bradyrhizobium japonicum* (Lorite MJ. Et al., Appl. Environ. Microbiol., 2000, vol. 66(5), p.1871-1876) (King GM et al., Appl. Environ. Microbiol. 2003, 69 (12), 7257-7265). Also in *Ralsotonia* bacteria which are aerobic hydrogen oxidizing bacteria, oxygen insensitive CODH exists (NCBI Gene ID: 4249199, 8019399).

As described above, bacteria having CODH widely exist. The obligately anaerobic acetogen of the present invention can be appropriately selected from such bacteria. For example, using a selective medium containing CO, CO/H₂ (gas mainly containing CO and H₂), or CO/CO₂/H₂ (gas mainly containing CO, CO₂ and H₂) as a sole carbon source and energy source, a bacterium having CODH that is usable as the obligately anaerobic acetogen of the present invention can be isolated in anaerobic conditions.

The obligately anaerobic acetogen of the present invention can be cultured by a well-known method as a culture method for obligatory anaerobic microorganisms. For example, the culture can be carried out under the anaerobic conditions by using carbon sources such as saccharides, acetic acid, ethanol, methanol, and glycerine.

The present invention includes a recombinant microorganism including the above-described obligately anaerobic acetogen into which a foreign gene encoding a target protein is introduced, and being capable of expressing the target protein. Preferably, the foreign gene is incorporated into a genome.

The method of introducing a gene (DNA) into the obligately anaerobic acetogen of the present invention may be selected appropriately depending on the kind of the obligately anaerobic acetogen, and the like. For example, a vector that can be introduced into the microorganism and can allow expression of the gene incorporated therein may be used.

For example, when the microorganism is a prokaryote such as a bacterium, as the vector, a vector that can self-duplicate or can be incorporated in chromosome in a host cell, and contains a promoter at the position allowing transcription of the inserted foreign gene can be used. For example, it is preferred to construct a series of structures including a promoter, a ribosome binding sequence, the above foreign gene, and a transcription termination sequence in the microorganism by using the vector.

In the case where the obligately anaerobic acetogen is a *Clostridium* bacterium (including related species such as *Moorella* bacteria), a shuttle vector pIMP1 between *Clostridium* bacterium and *Escherichia coli* (Mermelstein LD et al., Bio/technology 1992, 10, 190-195) may be used. The shuttle vector is a fusion vector of pUC9 (ATCC 37252) and pIM13 isolated from *Bacillus subtilis* (Projan SJ et al., J. Bacteriol. 1987, 169 (11), 5131-5139) and is retained stably in the *Clostridium* bacterium.

For introducing a plurality of kinds of target genes (DNA) into the obligately anaerobic acetogen of the present invention by using a vector, the genes may be incorporated in one vector, or incorporated in individual vectors. When a plurality of kinds of genes are incorporated into one vector, these genes may be expressed under a common promoter for these genes, or expressed under individual promoters. In order to express a plurality of genes in a form of a fusion protein, a fused gene in which a plurality of genes are linked to each other may be introduced.

The recombinant microorganism of the present invention can be cultured by a method well known as a culture method of an obligatory anaerobic microorganism. For example, it can be cultured using carbon sources such as saccharides, acetic acid, ethanol, methanol, and glycerin under anaerobic conditions.

The culture can be carried out using syngas. For example, when a gas component is used as a carbon source and an energy source, it is cultured, for example, in a nutrient condition including inorganic salts required for growth, and syngas. Preferably, the culture is carried out under a pressurized condition at about 0.2 to 0.3 MPa (absolute pressure). Thus, the assimilation property of the gas component is enhanced. Furthermore, for improving initial proliferation and attained cell density, small amounts of organic substances such as vitamins, yeast extract, corn steep liquor, and Bacto Tryptone, may be added.

When the recombinant microorganism of the present invention is cultured, a target protein encoded by the introduced foreign gene can be expressed. If necessary, it is possible to isolate the target protein from a cultured product.

### EXAMPLES

In the following section, the present invention will be described more specifically by way of Examples. However, the present invention is not limited to these Examples.

### (1) Construction of CRISPR-Cas9 and sgRNA expression vector for deletion of type I restriction enzyme

An artificially synthesized gene (6701 bp) of SEQ ID NO: 2 as a CRISPR-Cas9 and sgRNA expression gene cluster for deletion of type I restriction enzyme was constructed. The gene cluster includes Csn1 derived from *Streptococcus pyogenes* (endonuclease, GenBank: AAZ51387.1), each gene of a sgRNA expression cassette, and an upstream sequence (LHA, 1008 bp), a downstream sequence (RHA, 999 bp), a thl promoter (Pthl), an araE promoter (ParaE), and a fdx terminator (fdx terminator) of hsdR (type I restriction enzyme, GenBank: ADK13415.1) of *Clostridium ljungdahlii*, and further includes recognition sequences of NotI and XhoI at both terminals.

The artificially synthesized DNA (43 bp) of SEQ ID NO: 3 was cloned to the BamHI/SalI site of *Clostridium*/*E*. *coli* shuttle vector pCL2 to obtain pCL2-MS. Furthermore, a gene fragment of an artificially synthesized gene of SEQ ID NO: 2 that had been treated with a NotI/XhoI restriction enzyme was cloned to a NotI/XhoI site of pCL2-MS to obtain pCL2-TypeIKO (FIG. 1). In the cloning, *E*. *coli* JM109 was used. Similar to pCL2, pCL2-TypeIKO functions as a shuttle vector. The entire nucleotide sequence of pCL2-TypeIKO is shown in SEQ ID NO: 4.

### (2) Preparation of modified Clostridium ljungdahlii in which type I restriction enzyme activity is deleted

pCL2-TypeIKO prepared in the above (1) was introduced into *Clostridium ljungdahlii* (DSM13528/ATCC55383) by electroporation to obtain a recombinant (modified *Clostridium ljungdahlii*). As a control, similarly, pCL2-MS was introduced into *Clostridium ljungdahlii* (DSM13528/ATCC55383) to obtain a recombinant. The electroporation was carried out by a method recommended in Appl. Environ. Microbiol. 2013, 79(4): 1102-9. The genomic DNA of the obtained recombinant was extracted and subjected to sequencing to confirm that the type I restriction enzyme gene had been deleted from the genome.

### (3) Confirmation of improvement in transformation efficiency by modified Clostridium ljungdahlii

*Clostridium*/*E*. *coli* shuttle vector pCL2 was introduced into the type I restriction enzyme deleted recombinant (modified *Clostridium ljungdahlii*) obtained in the above (2) and wild-type *Clostridium ljungdahlii* (DSM13528/ATCC55383) by electroporation, and comparison of the transformation efficiency was carried out. As a result, when the wild-type *Clostridium ljungdahlii* (DSM13528/ATCC55383) was used as a host, the transformation efficiency was 1.0×10² cfu/µg, while the value was 1.0×10⁵ cfu/µg when the type I restriction enzyme deleted recombinant (modified *Clostridium ljungdahlii*) was used. The transformation efficiency was significantly improved.

From the above, it was shown that by using the modified *Clostridium ljungdahlii* in which the type I restriction enzyme activity had been deleted, the transformation efficiency was improved and transformation into *Clostridium ljungdahlii* was able to be carried out easily.

### (4) Confirmation of improvement in gene deletion efficiency by modified Clostridium ljungdahlii

With reference to Appl. Environ. Microbiol. 2013, 79(4): 1102-9, pUC-Δpta-ermC (SEQ ID NO: 5) including the upstream sequence and downstream sequence of a pta gene (CLJU_c12770) of C. *ljungdahlii*, and clarithromycin resistance gene ermC (GenBank: AB982225.1) was prepared. The pUC-Δpta-ermC was introduced into the type I restriction enzyme deleted recombinant (modified *Clostridium ljungdahlii*) obtained in the above (2) and the wild-type *Clostridium ljungdahlii* (DSM13528/ATCC55383) by electroporation, and comparison of the gene deletion efficiency was carried out. As a result, the gene deleted recombinant obtained by using the wild-type *Clostridium ljungdahlii* (DSM13528/ATCC55383) as a host was only one clone, while 13 clones were obtained in the case where the type I restriction enzyme deleted recombinant (modified *Clostridium ljungdahlii*) was used, in which the gene deletion efficiency was significantly improved.

From the above, it was shown that by using the modified *Clostridium ljungdahlii* in which the type I restriction enzyme activity had been deleted, the gene deletion efficiency was improved, and the genome gene of *Clostridium ljungdahlii* was able to be modified easily.

### (5) Confirmation of proliferation efficiency of modified Clostridium ljungdahlii

The type I restriction enzyme deleted recombinant (modified *Clostridium ljungdahlii*) obtained in the above (2) and the wild-type *Clostridium ljungdahlii* (DSM13528/ATCC55383) were cultured at 37°C under anaerobic conditions, respectively. Specifically, inoculation was carried out in 25 mL of ATCC 1754 medium (pH = 5.5, fructose was not contained), and a 100 mL-volume hermetically-sealable headspace vial vessel was charged with a mixed gas of CO/CO₂/H₂ = 33/33/34% (volume ratio), filled with the mixed gas at a gas pressure of 0.25 MPa (absolute pressure), hermetically sealed with an aluminum cap, and then shaking culture was conducted. As a result, in the type I restriction enzyme deleted recombinant (modified *Clostridium ljungdahlii*), proliferation that is the same level as in the wild-type *Clostridium ljungdahlii* (DSM13528/ATCC55383) was confirmed.

From the above it was shown that the type I restriction enzyme deleted mutation did not have an influence on the proliferation.

## Claims

1. A modified obligately anaerobic acetogen in which activity of at least one enzyme selected from the group consisting of a restriction enzyme, a modification enzyme, and a recognition enzyme constituting a type I restriction modification system enzyme is deleted or suppressed.

2. The obligately anaerobic acetogen according to claim 1, wherein the activity of at least the restriction enzyme is deleted or suppressed.

3. The obligately anaerobic acetogen according to claim 1, wherein a part or whole of a gene encoding at least one enzyme selected from the group consisting of the restriction enzyme, the modification enzyme, and the recognition enzyme is deleted.

4. The obligately anaerobic acetogen according to claim 3, wherein a part or whole of a gene encoding at least the restriction enzyme is deleted.

5. The obligately anaerobic acetogen according to any one of claims 1 to 4, wherein the activity of the restriction enzyme, the modification enzyme, or the recognition enzyme is deleted.

6. The obligately anaerobic acetogen according to any one of claims 1 to 5, being capable of proliferating using carbon monoxide or carbon dioxide as a sole carbon source.

7. The obligately anaerobic acetogen according to any one of claims 1 to 6, comprising a carbon monoxide dehydrogenase.

8. The obligately anaerobic acetogen according to any one of claims 1 to 7, having a function of synthesizing acetyl-CoA from methyl tetrahydrofolate, carbon monoxide, and CoA.

9. The obligately anaerobic acetogen according to any one of claims 1 to 8, being a bacterium.

10. The obligately anaerobic acetogen according to claim 9, being a *Clostridium* bacterium or a *Moorella* bacterium.

11. The obligately anaerobic acetogen according to claim 10, being *Clostridium ljungdahlii*.

12. The obligately anaerobic acetogen according to any one of claims 1 to 11, wherein the restriction enzyme is a protein including an amino acid sequence of SEQ ID NO: 1.

13. The obligately anaerobic acetogen according to claim 1, being *Clostridium ljungdahlii*,
wherein the activity of at least the restriction enzyme is deleted or suppressed, and
a part or whole of a gene encoding at least the restriction enzyme is deleted.

14. A recombinant microorganism comprising the obligately anaerobic acetogen according to any one of claims 1 to 13 into which a foreign gene encoding a target protein is introduced, and being capable of expressing the target protein.

15. The recombinant microorganism according to claim 14, wherein the foreign gene is incorporated into a genome.
